(19) Europäisches Patentamt
European Patent Office
Office européen des brevets

(11) Veröffentlichungsnummer: **0 513 560 B1**

(12) **EUROPÄISCHE PATENTSCHRIFT**

(45) Veröffentlichungstag der Patentschrift: **20.09.95**

(51) Int. Cl.$^{6}$: **G01N 33/533**, C09B 69/10

(21) Anmeldenummer: **92106783.1**

(22) Anmeldetag: **21.04.92**

# (54) Polymere Farbstoffe und deren Verwendung.

(30) Priorität: **03.05.91 DE 4114482**

(43) Veröffentlichungstag der Anmeldung:
**19.11.92 Patentblatt 92/47**

(45) Bekanntmachung des Hinweises auf die Patenterteilung:
**20.09.95 Patentblatt 95/38**

(84) Benannte Vertragsstaaten:
**CH DE FR GB IT LI SE**

(56) Entgegenhaltungen:
**EP-A- 0 244 929** **EP-A- 0 342 052**
**EP-A- 0 361 229** **EP-A- 0 361 229**
**FR-A- 2 295 426** **FR-A- 2 295 426**

(73) Patentinhaber: **BAYER AG**
**D-51368 Leverkusen (DE)**

(72) Erfinder: **Heiliger, Ludger, Dr.**
**Carl-Rumpff-Strasse 8**
**W-5090 Leverkusen 1 (DE)**
Erfinder: **Siegmund, Hans-Ulrich, Dr.**
**Roonstrasse 100**
**W-4150 Krefeld 1 (DE)**
Erfinder: **Hugl, Herbert, Dr.**
**Gemarkenweg 9**
**W-5060 Bergisch Gladbach 2 (DE)**
Erfinder: **Löbberding, Antonius, Dr.**
**Am Rohm 105**
**W-5600 Wuppertal 1 (DE)**
Erfinder: **Kuckert, Eberhard, Dr.**
**Am Scherfenbrand 67**
**W-5090 Leverkusen 1 (DE)**
Erfinder: **Bömer, Bruno, Dr.**
**Max-Planck-Strasse 53**
**W-5060 Bergisch Gladbach 2 (DE)**
Erfinder: **Böcker, Thomas, Dr.**
**Vohwinkelallee 28**
**W-4000 Düsseldorf 1 (DE)**
Erfinder: **Franke, Günter, Dr.**
**Landrat-Trimborn-Strasse 60**
**W-5653 Leichlingen 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 513 560 B1

## Beschreibung

Die vorliegende Erfindung betrifft polymere Farbstoffe, d.h. polymer-gebundene Farbstoffe, ein Verfahren zu deren Herstellung und deren Verwendung beispielsweise als Markierungssubstanzen in Analysenverfahren. Polymere Farbstoffe enthalten verknüpfbare funktionelle Gruppen und sind unter üblichen Analysenbedingungen wasserlöslich. Verantwortlich für diese Wasserlöslichkeit ist normalerweise der Polymeranteil. Die Farbstoffe an sich sind oft wasserunlöslich.

Bekannte Markierungssubstanzen, die in biologischen Testsystemen einsetzbar sind, haben häufig den Nachteil schlechter Arbeitssicherheit und der Handhabbarkeit nur in speziell ausgestatteten Labors (Radioaktivität) oder der mangelhaften Stabilität, z.B. bei der Enzymmarkierung.

Fluoreszierende Polymere und deren Herstellung mittels vorzugsweise Aminogruppen enthaltenden Farbstoffen sind bekannt. In der US-PS 4 166 105 werden Reagenzien beschrieben, die sich zum Nachweis spezifischer Reaktanten, z.B. Antigene, eignen und aus einem an einen Antikörper gebundenen Polymeren, welches eine Vielzahl von Farbstoffmolekülen enthält, bestehen.

Die Farbstoffpolymeren besitzen endständige funktionelle Gruppen, die zur Verknüpfung mit dem Protein und eine Vielzahl anderer funktioneller Gruppen, die zur Bindung der Farbstoffmoleküle genutzt werden. Als geeignete Rückgrat-Polymere werden z.B. Polyethylenimine, Polylysin, Polyamide und niedermolekulare Polycarbonsäuren genannt.

Im ersten Beispiel des genannten Patents wird die Synthese eines polymeren Farbstoffs aus Polyethylenimin und Fluoresceinisothiocyanat (FITC) beschrieben, der 70 Farbstoffmoleküle je Molekül Polyethylenimin enthält. Gemäß Beispiel 7 wird die Quantenausbeute eines derartigen polymeren Farbstoffs mit 80 gebundenen Fluoresceineinheiten bestimmt. Diese beträgt nur 4 %. Daraus folgt, daß dieses Polymer bei hundertfachem Molekulargewicht nur etwa dreimal so stark fluoresziert wie monomeres FITC.

In der DE-OS 3 921 498 werden fluoreszierende polymere Reagenzien beschrieben, die hergestellt werden durch Umsetzung von Dicarbonsäureanhydrid-Gruppen enthaltenden Co-Polymeren mit Aminogruppen enthaltenden Farbstoffen. Gemäß Beispiel 4 sollen solche Reagenzien hohe Quantenausbeuten von über 60 % erbringen.

Trotz dieser hohen Quantenausbeuten sind die molaren Amplifikationen, verglichen mit den eingesetzten monomeren Farbstoffen, noch nicht befriedigend, da die Molmassen des wasserlöslichen Gerüstpolymeren noch zu niedrig sind. Der Amplifikationsgrad des Systems wird durch die Fluoreszenzintensität bestimmt, die sich ihrerseits aus dem Produkt der molaren Extinktion und der Fluoreszenzquantenausbeute berechnen läßt.

Um eine hohe Amplifikation gegenüber dem eingesetzten monomeren Farbstoff zu erreichen, müssen daher beide Faktoren (molare-Extinktion und Fluoreszenzquantenausbeute) möglichst groß sein.

Bei den bisher beschriebenen polymeren Systemen ist jedoch wenigstens einer dieser beiden Faktoren zu gering, um die gewünschte hohe Amplifikation zu erreichen.

Aus der EP-A 244 929 sind stabile, schnell reagierende, fluoreszente, polymere Indikatoren für die Bestimmung der Konzentration einer in einem wäßrigen Medium gelösten Substanz bekannt, die ein oranisches Polymer enthalten, das mehrere fluoreszente, organische Substituenten aufweist, die gleich oder verschieden sein können und an das Polymer über Ester- oder Amidbindungen gebunden sind.

Die EP-A 342 052 beschreibt ein Monomer zur Herstellung eines gefärbten Polymers, wobei das Monomer erhalten wird durch Reaktion mit einem Farbstoff und so ein gefärbtes Monomer entsteht. Das gefärbte Monomer, das auf Acrylat basieren und als Farbstoff Reaktivblau 2 oder Reaktivrot 120 enthalten kann, wird polymerisiert und/oder mit einem anderen gefärbten und/oder ungefärbten Monomer copolymerisiert, um ein gefärbtes Polymer herzustellen. Das gefärbte Polymer wird verwendet, um biologisch aktive Substanzen zu reinigen, beispielsweise durch Affinitätschromatographie oder Elektrophorese.

Es wurden nun wasserlösliche polymere Farbstoffe gefunden, die dadurch gekennzeichnet sind, daß sie den allgemeinen Aufbau der Formel (I) aufweisen

$$[A]_a\text{-}[B]_b\text{-}[C]_c\text{-}[D]_d \qquad (I),$$

worin

A einen gegebenenfalls vorhandenen wasserlöslich machenden Baustein,

B ein über eine Ester-, Säureamid-, Urethan-, Harnstoff- und/oder Thioharnstoff-Gruppierung kovalent gebundenes Fluoreszenzfarbstoffmolekül,

C einen zweiten Fluoreszenzfarbstoff, der über eine Ester-, Säureamid-, Urethan-, Harnstoff-und/oder Thioharnstoff-Gruppierung kovalent gebunden und nach Anregung ein Licht in einem Wellenlängenbereich emitiert, das um ± 30 nm von der Wellenlänge abweicht, bei der der jeweilige

Fluoreszenzfarbstoff B seine maximale Absorption besitzt und

D einen Monomerbaustein, der eine kovalente Verknüpfung zu einem Antikörper, Antigen Hapten, einer Nucleinsäure oder einem geeignet funktionalisierten Oligonucleotid und gegebenenfalls zu den Komponenten B und/oder C ermöglicht,

bedeutet und

a,b,c und d die gew.-%igen Anteile der Komponenten A, B, C und D darstellen, die zusammen 100 Gew.-% ergeben.

Die wasserlöslich machenden Bausteine A können ionisch oder nichtionisch sein. Es kann sich dabei beispielsweise um Acrylsäure, Methacrylsäure, Acrylamid, Methacrylamid oder Derivate davon handeln. Als Derivate kommen z.B. in Frage: 2-Acryloylamino-2-methyl-propansulfonsäure, Dialkylamino-alkyl-(meth)-acrylate und Dialkylamino-alkyl-(meth)-acrylamide wie Dimethylamino-ethyl-methacrylat, Dimethylamino-propyl-acrylamid und die sich von solchen (Meth)acrylaten und (Meth)acrylamiden ableitenden quarternierten Verbindungen. Ferner kommen beispielsweise in Frage: N-Vinylpyrrolidon, N-Vinylpiperidon, N-Vinylcaprolactam, N-Vinylformamid, N-Vinylacetamid, N-Vinyl-N-methyl-acetamid und N-Vinyl-O-methylurethan.

Als Fluoreszenzfarbstoffe B und C kommen beispielsweise in Frage Cumarine der Formel (II)

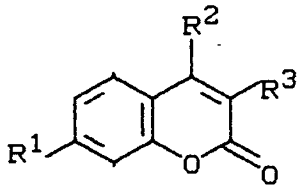

(II),

in der

$R^1$ für O-Alkyl, $N(Alkyl)_2$, NH-Alkyl, $NH-SO_2$-Alkyl, O-Trimethylsilyl oder $NH-SO_2$-Aryl,

$R^2$ für Wasserstoff, Cyano, Chlor, Hydroxy, Alkyl oder Aryl und

$R^3$ für Phenyl oder Hetaryl stehen.

Alkyl bedeutet dabei vorzugsweise $C_1$- bis $C_6$-Alkyl, Aryl vorzugsweise Phenyl, Hetaryl vorzugsweise (Benz)thiazolyl

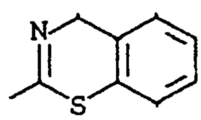

$R^1$ kann außerdem

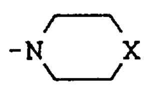

bedeuten, wobei X für Sauerstoff,

$N-C_1$- bis $C_4$-Alkyl oder $(CH_2)_n$ steht, worin n = 0 oder 1 sein kann.

Weiterhin geeignet sind Cumarine der Formel (III)

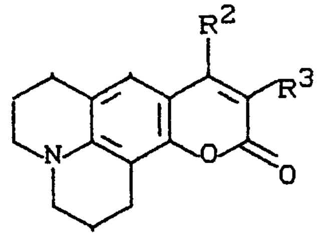

(III),

worin

$R^2$ und $R^3$ die bei Formel (II) angegebene Bedeutung haben.

Vorzugsweise enthalten die Cumarine der Formeln (II) und (III) an einem der Substituenten $R^1$, $R^2$ und $R^3$ eine funktionelle Gruppe für die Verknüpfung des Farbstoffs mit einem Monomerbaustein D oder dem daraus zugänglichen Polymer. $NH_2$- oder OH-Gruppen sind dafür besonders geeignet.

Ebenfalls geeignet sind Carbostyrile der Formel (IV)

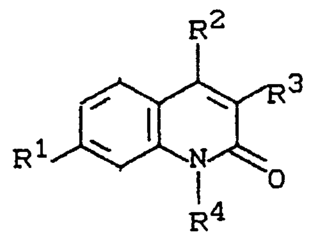

(IV),

worin

$R^1$, $R^2$ und $R^3$ die bei den Cumarinen (siehe Formeln (II) und (III)) angegebenen Bedeutungen haben und

$R^4$ für Alkyl, vorzugsweise für $C_1$- bis $C_6$-Alkyl steht.

Auch hier enthält einer der Substituenten vorzugsweise eine funktionelle Gruppe für die Verknüpfung mit einem Monomerbaustein D oder dem daraus zugänglichen Polymer.

Weiterhin sind Pyrazoline der Formel (V) geeignet

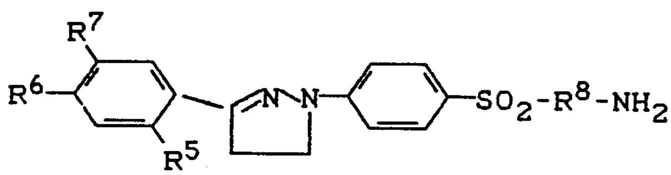

(V),

worin

$R^5$ für Wasserstoff oder Methyl,

$R^6$ und $R^7$ unabhängig voneinander für Wasserstoff oder Chlor und

$R^8$ für Alkylen,

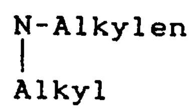

oder Alkylen-O-Alkylen stehen,

wobei Alkyl und Alkylen beispielsweise $C_1$- bis $C_6$-Alkyl bzw. $C_1$- bis $C_6$-Alkylen bedeuten kann.

Auch geeignet sind Naphthalimide der Formel (VI)

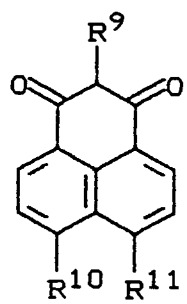

(VI),

worin

$R^9$ für Alkyl und

$R^{10}$ und $R^{11}$ unabhängig voneinander für Wasserstoff, O-Alkyl oder $N(Alkyl)_2$ stehen,

wobei Alkyl vorzugsweise jeweils $C_1$- bis $C_6$-Alkyl bedeutet und einer der Reste $R^9$, $R^{10}$ oder $R^{11}$ eine $NH_2$-Gruppe zur Verknüpfung mit einem Monomerbaustein D oder dem daraus zugänglichen Polymer trägt.

Ebenfalls geeignet sind Pyrene der Formel (VII)

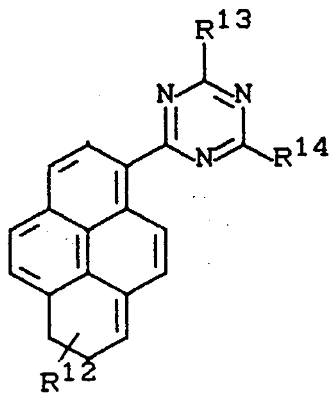

(VII),

worin

$R^{12}$ für Wasserstoff oder $SO_3H$ und

$R^{13}$ und $R^{14}$ unabhängig voneinander für O-Alkyl oder $N(Alkyl)_2$ stehen,

wobei Alkyl vorzugsweise $C_1$- bis $C_6$-Alkyl bedeutet und einer der Reste $R^{13}$ oder $R^{14}$ eine $NH_2$-Gruppe zur Verknüpfung mit einem Monomerbaustein D oder dem daraus zugänglichen Polymer trägt.

Es kommen auch Fluoresceine der Formel (VIII)

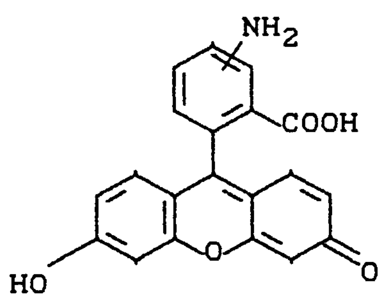

(VIII),

und Rhodanine der Formel (IX) in Frage

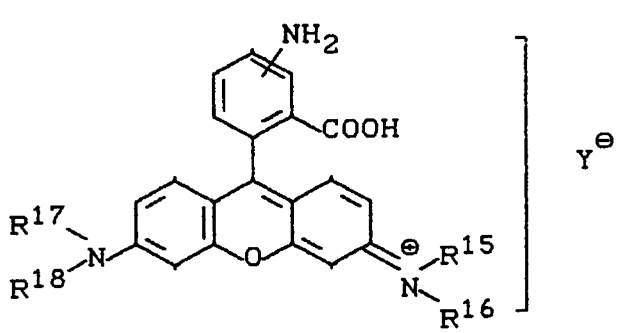

(IX),

worin

$Y^{\ominus}$ ein farbloses Anion, z.B. $Cl^{\ominus}$, $Br^{\ominus}$, $I^{\ominus}$, $HSO_4^{\ominus}$,

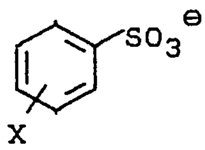

$X$ = Cl, Br, I, $CH_3$
bedeutet und

$R^{15}$ bis $R^{18}$ unabhängig voneinander für Alkyl oder

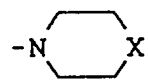

stehen,
wobei Alkyl vorzugsweise $C_1$- bis $C_6$-Alkyl bedeutet und X für Sauerstoff, N-$C_1$- bis $C_4$-Alkyl oder $(CH_2)_n$ steht, worin n Null oder 1 sein kann.

$\overset{\oplus}{N}R^{15}R^{16}$ und/oder $NR^{17}R^{18}$ können auch zusammen mit dem aromatischen Ring, an den sie gebunden sind, ein polycyclisches System bilden, z.B. ein System der Formel (X) oder (XI).

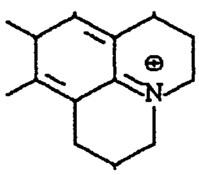
(X)

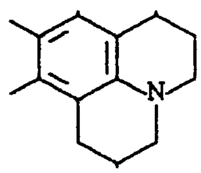
(XI)

Diese und weitere geeignete Farbstoffe sind bekannt (siehe z.B. "The Chemistry of Synthetic Dyes", Vol, V, Academic Press (1971) und "Fluorescent Whitening Agents", G. Thieme Verlag Stuttgart (1975)).

Die Monomerbausteine D enthalten reaktive oder aktivierbare Gruppen, welche die kovalente Bindung zu einem Protein und gegebenenfalls den Komponenten B und/oder C ermöglichen. Derartige Gruppen können beispielsweise Säurehalogenid-, Imidester-, Benztriazolyl-, Isocyanato-, Isothiocyanato-, Oxiran oder Diimid-Gruppen sein. Bei dem polymerisierbaren Teil von D kann es sich beispielsweise um einen Acryl-, Methacryl-, Vinyl- oder Styryl-Rest handeln. D ist vorzugsweise (Meth)Acrylsäurechlorid, (Meth)Acrylsäure-N-hydroxy-succinimidester, (Meth)Acrylsäure-N-hydroxy-phthalimidester, N-(Meth)acryloylbenztriazol,3- oder 4-Isothiocyanatophenyl-(meth)acrylat, 2-Isocyanatoethyl(meth)acrylat, Isocyanatoisopropenylbenzol, Isopropenyl-$\alpha$-$\alpha$-dimethylbenzylisocyanat, Vinyloxiran oder eine Kombination aus (Meth)Acrylsäure mit Carbodiimiden.

Bei dem Protein, an das D kovalent anbindbar ist, kann es sich beispielsweise um einen Antikörper, ein Antigen, ein Hapten oder um Nucleinsäuren handeln.

a liegt beispielsweise zwischen 0 und 90 Gew.-%, b beispielsweise zwischen 5 und 50 Gew.-%, c beispielsweise zwischen 5 und 99 Gew.-% und d beispielsweise zwischen 0,01 und 10 Gew.-%. Vorzugsweise ist a = Null, d.h. gesonderte wasserlöslich machende Bausteine sind nicht vorhanden. Dann ergibt die Summe b + c + d 100 Gew.-% und b liegt vorzugsweise zwischen 10 und 40 Gew.-%, c vorzugsweise zwischen 59 und 89,95 Gew.-% und d vorzugsweise zwischen 0,05 und 5 Gew.-%.

Von den erfindungsgemäßen polymeren Farbstoffen der Formel (I) sind solche bevorzugt, bei denen C bedeutet:

Einen im Sinne der oben angegebenen Definition zu B komplementären Fluoreszenzfarbstoff, der mindestens eine Carbon- oder Sulfonsäuregruppe und/oder mindestens eine Carboxylat- oder Sulfonatgruppe enthält, wobei C über eine Ester-, Säureamid-, Urethan-, Harnstoff-und/oder Thioharnstoff-Gruppierung kovalent gebunden ist.

Ein besonders bevorzugtes Beispiel für einen Farbstoff B und einen dazu komplementären Farbstoff C ist Rhodamin B als Farbstoff B und Fluorescein als Farbstoff C.

Bei besonders bevorzugten erfindungsgemäßen polymeren Farbstoffen der Formel (I) steht B für ein über eine Ester-, Säureamid-, Urethan-, Harnstoff- und/oder Thioharnstoff-Gruppierung gebundenes Cumarin der Formel (II) und D für (Meth)Acrylsäurechlorid, (Meth)Acrylsäure-N-hydroxy-succinimidester, (Meth)-Acrylsäure-N-hydroxy-phthalimidester, N-(Meth)acryloylbenztriazol, 3-oder 4-Isothiocyanatophenyl(meth)-acrylat, 2-Isocyanatoethyl(meth)acrylat, Isocyanatostyrol, Isocyanatoisopropenylbenzol, Isopropenyl-$\alpha,\alpha$-dimethylbenzylisocyanat, Vinyloxiran oder eine Kombination aus (Meth)Acrylsäure mit Carbodiimiden, a für Null, b für 12 bis 35 Gew.-%, c für 62 bis 85 Gew.-% und d für 0,07 bis 4,5 Gew.-%.

Erfindungsgemäße polymere Farbstoffe der Formel (I) können beispielsweise durch eine an sich bekannte radikalische Polymerisation der Komponenten A bis D hergestellt werden. Geeignete Radikalbildner sind beispielsweise Peroxide und Azoverbindungen wie Benzoylperoxid, Azobisisobutyronitril und geeignete Lösungsmittel beispielsweise Dimethylformamid und Dimethylsulfoxid.

Farbstoffmoleküle B und C können z.B. durch Copolymerisation entsprechender Farbstoffmonomeren, beispielsweise (Meth)Acrylsäureester oder (Meth)Acrylamide davon, oder durch Umsetzung von vorzugsweise Aminogruppen enthaltenden Farbstoffen mit Reaktivgruppen des polymeren Rückgrats in polymeranaloger Reaktion eingeführt werden.

Als reaktive oder aktivierbare Gruppen kommen für D beispielsweise Säurehalogenid-, Imidester-, Benztriazolyl-, Isocyanato-, Isothiocyanato-, Oxiran- oder DiimidGruppen in Frage. Wenn D Säurehalogenidgruppen enthält ist es vorteilhaft Protonenfänger einzusetzen, z.B. tertiäre Amine. Als Lösungsmittel für die Einführung der Farbstoffe B und C und/oder aromatische Bausteine C wie auch für die Aktivierung der für die Verknüpfung mit biologischem Material einpolymerisierten Reaktivgruppen kommen solche in Frage, die inert gegenüber den Reaktivgruppen sind, z.B. Dimethylformamid, Dimethylsulfoxid, Dimethylacetamid und Acetonitril.

Die Aktivierung gegen Hydrolyse empfindlicher Reaktivgruppen für die Verknüpfung mit biologischen Materialien kann beispielsweise mit Aminoalkoholen der Formel $H_2N\text{-}R^{24}\text{-}OH$ mit $R^{24}$ = $C_1$- bis $C_{12}$-Alkylen entweder vor oder nach der Polymerisation erfolgen, wodurch OH-funktionelle Gruppen entstehen, die ihrerseits z.B. durch Überführung in die Trifluormethansulfonyl-, Methansulfonyl-, Trifluoracetyl-, Benzolsulfonyl-, Benzoesäure-3-sulfonyl- oder p-Toluolsulfonyl-Gruppe aktiviert werden können.

Ferner können zu diesem Zweck Monomere eingebaut werden, die bereits eine aliphatische Alkoholgruppe enthalten, wie Hydroxyalkyl(meth)acrylate, z.B. Hydroxyethyl(meth)acrylat, Hydroxypropyl(meth)-acrylat oder Butandiolmono-(meth)acrylat, die ebenso aktiviert werden können.

Wenn auf wäßrige Reaktionsmedien bei der Verknüpfung des biologischen Materials mit dem polymeren Farbstoff verzichtet werden kann oder die Reaktivgruppe gegen Hydrolyse stabil ist, können die Farbstoffpolymeren direkt ohne vorherige Aktivierung mit dem biologischen Material umgesetzt werden.

Nach der Durchführung der Aktivierung kann der polymere Farbstoff nach an sich bekannten Methoden isoliert werden, z.B. durch Abdampfen des Lösungsmittels und/oder durch Ausfällen des polymeren Farbstoffs in einem geeigneten organischen Medium.

Die Abtrennung eines eventuellen Überschusses an Reagenzien kann entweder beim Ausfällen des polymeren Farbstoffs, durch erneutes Umfällen oder im Falle wasserlöslicher Reagenzien auch durch Dialyse oder Ultrafiltration erfolgen.

Anschließend kann der gereinigte polymere Farbstoff getrocknet und so die erfindungsgemäßen polymeren Farbstoffe erhalten werden.

Das aktivierte Farbstoffpolymer kann dann in wäßriger Lösung mit dem biologischen Substrat (z.B. Antikörper oder geeignet funktionalisiertes Oligonucleotid) umgesetzt werden. Die erhaltene Mischung kann entweder direkt in Tests (z.B. Immunoassay oder Gensondentest) eingesetzt werden oder auch nach vorheriger Reinigung.

Geeignet funktionalisierte Oligonucleotide sind bekannt. Darunter werden z.B. Oligonucleotide verstanden, die über einen internen Spacer Amino-, Mercapto- oder Hydroxyfunktionen enthalten.

Eine für die Leistungsfähigkeit solcher Tests entscheidende Größe ist die Empfindlichkeit. Sie wird bei den meisten derzeit üblichen Testverfahren durch Einsatz radioaktiver Markierungssubstanzen erreicht.

Diese Methode hat in der praktischen Durchführung aber viele gravierende Nachteile (Strahlengefährdung, Zersetzlichkeit der Substanzen, schwierige Abfallentsorgung, Spezialausrüstung der Labors, spezielle Ausbildung des Personals), die eine Ausdehnung dieses an sich vorteilhaften leistungsfähigen Tests zu Routine-verfahren bisher verhindert haben (siehe z.B. WO 88-02784 und Pharmacia vom 21.4.1988).

Es sind mehrere Versuche unternommen worden, die radioaktive Markierung durch eine problemlose Farbstoffmarkierung zu ersetzen. Damit umgeht man die Nachteile der radioaktiven Markierung, die erzielbaren Empfindlichkeiten sind aber für viele Tests nicht ausreichend (siehe z.B. Nucleic Acids Res. 16, 4957 (1988)).

Hier bieten die erfindungsgemäßen polymeren Farbstoffe den Vorteil einer gesteigerten Empfindlichkeit ohne Verwendung von Radioaktivität.

Die erfindungsgemäßen polymeren Farbstoffe haben normalerweise mittlere Molekulargewichte in der Größenordnung von $\overline{M}_n$ = 2 x $10^3$ bis 5 x $10^6$ Dalton. Bevorzugt sind Molekulargewichte von etwa $10^4$ bis $10^6$ Dalton.

Unter üblichen Analysenbedingungen sind die polymere Farbstoffe in wäßrigen Medien zu mindestens 0,1 %, vorzugsweise zu mindestens 1 % löslich.

Übliche Analysenbedingungen sind dabei solche, wie sie in biologischen Tests, insbesondere bei Bindungsanalysenverfahren wie Immunoassays oder Gensonden-Tests vorkommen. Diese Bedingungen umfassen beispielsweise Temperaturen bis zu etwa 70 °C und pH-Werte zwischen etwa 3 und 11. Bei besonderen Tests kann von diesen Werten abgewichen werden. Wesentlich ist die Wasserlöslichkeit der polymeren Farbstoffe, weil dadurch der Einsatz der Farbstoffe in biologischen Analysenverfahren möglich ist.

Die erfindungsgemäßen polymeren Farbstoffe zeigen eine sehr viel höhere Fluoreszenzintensität als bekannte polymere Farbstoffe, da mit ihnen bei hohen molaren Extinktionen gleichzeitig noch hohe Quantenausbeuten erzielt werden können. Häufig erreichen erfindungsgemäße polymere Farbstoffe Amplifikationsgrade von über 100. (Siehe Bsp. 6).

Beispiel 1

Herstellung von polymerisierbaren Farbstoffmonomeren Cumarin Ib

Zu 4 g Cumarin Ia ($R_1$ = $NEt_2$, $R_2$ = H,

$R_3$ = 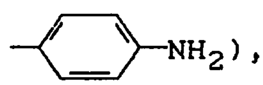

gelöst in 65 ml $CH_2Cl_2$, werden 1,35 g Acryloylchlorid, gelöst in 4 ml $CH_2Cl_2$, unter $N_2$-Atmosphäre bei 0 °C zugetropft. Danach wird auf Raumtemperatur erwärmt und 2h nachgerührt, der entstandene Niederschlag abgesaugt, mit $CH_2Cl_2$ gewaschen und bei Raumtemperatur im Hochvakuum getrocknet.
Die Ausbeute an Cumarin Ib ist quantitativ.

Beispiel 2

Herstellung von polymerisierbaren Farbstoffmonomeren Cumarin IIb

Zu 4 g Cumarin IIa ($R_1$ = $C_6H_5$—$SO_2$-NH-,
$R_2$ = H,

$R_3$ = 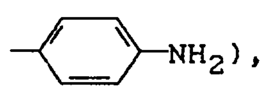

gelöst in 50 ml DMAC, werden 0,9 g Acryloylchlorid, gelöst in 5 ml DMAC , unter $N_2$-Atmosphäre bei 0 °C zugetropft. Nach der Hälfte des Zutropfens wird gleichzeitig 1,03 g Triethylamin, in 5 ml DMAC gelöst, zudosiert. Nach beendetem Zutropfen wird auf Raumtemperatur erwärmt und 2h nachgerührt, der entstandene Niederschlag abfiltriert, das Filtrat im Hochvakuum eingeengt und aus Ether kristallisiert.
Ausbeute an Cumarin IIb 85 %.

Beispiel 3

Herstellung von polymerisierbaren Comonomer Naphthylacrylat

4 g $\beta$-Naphthol werden unter den in Beispiel 2 beschriebenen Bedingungen mit 2,48 g Acryloylchlorid und 2,77 g Triethylamin reagiert. Ausbeute 78 %.

Beispiel 4

Herstellung von polymerisierbaren Reaktivgruppen

5 g Isocyantoethylmethacrylat, gelöst in 5 ml $CH_2Cl_2$, werden zu 3,77 g Aminohexanol, in 45 ml $CH_2Cl_2$ gelöst, bei 0 °C zugetropft, danach wird auf Raumtemperatur erwärmt und 1h nachgerührt. Das $CH_2Cl_2$ wird im Hochvakuum abrotiert und das Rohprodukt aus Ether kristallisiert. Ausbeute quantitativ.

Beispiel 5

Herstellung von polymeren Farbstoffen

1 g Cumarin IIb aus Beispiel 2, sowie Natrium-2-Acrylamido-2-methylpropansulfonat (Na-AMPS) oder Natrium-p-styrylsulfonat (Na-PSS) oder Methacrylsäure (MAA), Naphthylacrylat (Bsp. 3) und 12 mg AIBN (Gesamtmonomereinwaage 4 g, Zusammensetzung in Tabelle 1) werden in 25 ml DMSO eingetragen, die Apparatur evakuiert, mit $N_2$ begast, der Vorgang 3 mal wiederholt, die Lösung auf 65 °C erhitzt und 15h reagiert. Die Reaktionslösung wird in 200 ml Aceton eingetropft, der entstandene Niederschlag abfiltriert und getrocknet. Das Rohpolymer wird einer Ultrafiltration (Cutoff 10.000 Dalton) unterworfen.
Ausbeute 70 - 80 %.

Tabelle 1

| Vergleich der Quantenausbeuten von Farbstoffpolymeren mit aliphatischen und aromatischen Comonomeren | | | | | | |
|---|---|---|---|---|---|---|
| | A | B | C | D | E | F |
| Na-PSS | - | - | 75% | - | 50% | - |
| Na-AMPS | - | 75% | - | 50% | - | - |
| MAA | 75% | - | - | - | - | - |
| Cumarin IIb | 25% | 25% | 25% | 25% | 25% | 100%* |
| Naphthylacrylat | - | - | - | 25% | 25% | - |
| Quantenausbeute[1] | 0,23[1] | 0,15[1] | 0,5[1] | 0,25[1] | 0,5[1]<br>0,39[2] | 0,3[3]<br>0,41[2] |

* Monomer aus Bsp. 2
[1] in $H_2O$ bei pH 9
[2] in DMSO bei pH 9
[3] in $H_2O$/pH 9 nur teilweise gelöst

Beispiel 6

Abhängigkeit des Amplifikationsgrads von der Molmasse des Polymers

Bei Polymer $C_2$ wurde wie bei Polymer C aus Beispiel 5 verfahren, nur statt 20 ml DMSO 12 ml DMSO zur Polymerisation verwendet.

Die Molmassen und deren Verteilung wurden mittels wäßriger Gelpermationschromatographie, gekoppelt mit Kleinwinkellichtstreuung (LALLS), ermittelt.

Die Prozentanteile des Fluorophors in den Polymeren wurden durch die Formel

$$\frac{\text{Extinktion Polymer pro Gramm}}{\text{Extinktion Cumarin IIb pro Gramm}} \cdot 100\ \%$$

ermittelt.

| | Farbstoff % | $M_n$ | $M_w$ | $U = \frac{M_w}{M_n} - 1$ | Amplifikationsgrad |
|---|---|---|---|---|---|
| Polymer C (aus Bsp. 5) | 22 | 108.000 | 259.000 | 1,39 | 50,8 |
| Polymer $C_2$ | 22 | 337.000 | 787.000 | 1,33 | 158,5 |

Beispiel 7

Herstellung eines Polymers mit zwei zueinander komplementären Fluoreszenzfarbstoffen

In 8 ml Dimethylacetamid wurden 2 g Hydroxyethylmethacrylat gelöst, 20 mg AIBN zugegeben, der Reaktionskolben evakuiert, mit Reinststickstoff begast, der Vorgang 3 mal wiederholt, auf 65 °C erhitzt und 15 h reagieren gelassen. Nach Erkalten wurden der Rohlösung 10 ml DMSO und 10 mg Tetramethylrhodaminisothiocyanat zugegeben und 6 h bei 80 °C weitergerührt. Dann wurden 250 mg Fluoresceinisothiocyanat zugegeben und weitere 15 h bei 80 °C gerührt. Die Rohlösung wurde in Essigsäureethylester gefällt, abgesaugt und 2 mal in Ethanol/Ether umgefällt und im Hochvakuum getrocknet.

Das Rohpolymer wurde wie in Beispiel 5 beschrieben ultrafiltriert.

Das Emissionsspektrum des Polymeren, in Wasser-Methanol bei pH 11 gelöst, angeregt bei 500 nm, d.h. dem Extinktionsmaximum von Fluorescein, zeigte eine breite Schulter bei 575 nm, d.h. der Emmissionswellenlänge des Rhodamins, deren Anteil 27 % Quantenausbeute ausmachte. (anteilig errechnet aus dem Gesamtflächenintegral entsprechend einer Gesamtquantenausbeute von 73 %). Es fand demgemäß eine effiziente Energieübertragung von Fluorescein zu Rhodamin statt, wodurch letzteres sensibilisiert wurde. Die gleiche Lösung, angeregt bei 549,4 nm, d.h. direkte Anregung des Rhodamins im Extinktionsmaximum zeigte eine Emission, deren Maximum bei 574,7 nm und Quantenausbeute bei 30 % lag.

Beispiel 8

Synthese von verknüpfbaren polymeren Farbstoffen

Es wurde wie bei Polymer C aus Beispiel 5 verfahren, wobei zusätzlich 200 mg Reaktivmonomer aus Beispiel 4 zugesetzt wurde. Die DMSO-haltige Polymerlösung wurde nach der Reaktion mit Ig

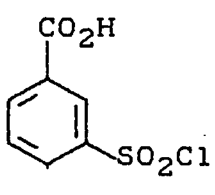

3-(Chlorsulfonyl)-benzoesäure und nach 30 Minuten mit 0,5 g Triethylamin versetzt, 1h bei Raumtemperatur reagieren lassen und wie in Beispiel 5 beschrieben, gefällt und ultrafiltriert.

Beispiel 9

Es werden 100 µg des Aminolinkoligonukleotids mit der Sequenz CTC GGA TCC CAT CTT CTC CCC TGA GTC TGT (Synthese gemäß N. D. Sinha u. R. M. Cook, Nucleic Acids Research 16, 2659 (1988) in 300 µl Carbonatpuffer (pH = 9) gelöst und mit einem Überschuß polymerem Fluoreszenzfarbstoff gemäß Beispiel 8 in 200 µl Carbonatpuffer versetzt. Die Reaktion erfolgt über 60h bei Raumtemperatur. Die Aufarbeitung erfolgt durch Gelfiltration an Biorad-Bio-Gel® P 4 oder durch reversed phase HPLC an RP 18 mit Triethylammoniumacetat/Acetonitril als Eluenten.

Beispiel 10

100 $\mu$g des Aminolinkoligonukleotids der Sequenz AT CTA CTG GCT CTT TTT TTT TTT TTT TTT TTT TTT TTT TTT T werden in 200 $\mu$l Carbonatpuffer gelöst (pH = 9) und mit einem Überschuß polymerem Fluoreszenzfarbstoff gemäß Beispiel 7 in 300 $\mu$l Carbonatpuffer versetzt und für 60h bei Raumtemperatur gerührt. Zur Aufarbeitung wird das gesamte Reaktionsgemisch auf eine Poly A-Sepharose 4B-Säule (5 ml) (Pharmacia) aufgetragen. Mit dem Auftragepuffer (A) (s. u. 20 ml) wird der nichtgebundene Farbstoff heruntergewaschen, dann wird mit dem Elutionspuffer (B) (s. u. 30 ml) gewaschen und schließlich das Kopplungsprodukt mit Elutionspuffer (C) (s. u. 40 ml) heruntergewaschen.

Auftragepuffer (A): 1,513 g Tris, 10,23 g NaCl, 0,56 g EDTA in 750 ml Wasser lösen und mit Formamid auf 1 Liter auffüllen.

Elutionspuffer (B): In 100 ml Auftragepuffer A 7,45 g KCl lösen.

Elutionspuffer (C): 3,7 g KCl und 50 ml Formamid mit Auftragepuffer A auf 100 ml auffüllen.

Bei der Verwendung der so markierten Aminolink-Oligonukleotide in DNA-Probe-Tests wird eine erhöhte Sensitivität verglichen mit DNA-Probes, die mit monomeren Fluoreszenz-Farbstoffen markiert sind, erreicht.

Beispiel 11

Verwendung von polymeren Fluoreszenzfarbstoffen in Immunologischen Testsystemen

10 mg Polymer Farbstoff gemäß Beispiel 8 werden in 10 ml Carbonat-Bicarbonatpuffer pH 9,0/0,5 molar gelöst. Dazu werden 5 mg Phosphodiesterase (aus Klapperschlangengift, Firma Sigma) in 5 ml Carbonatpuffer pH 9,0 (0,5 m) zugegeben, 4h gerührt bei Raumtemperatur über Nacht im Kühlschrank weiterreagieren lassen. Danach wird mit ln NaOH auf pH 11 gestellt und die Rohlösung chromatographiert (Sephacryl® 5-500 d. Firma Pharmacia in Carbonatpuffer 0,02 m, pH 11, Ø d. Säure 16 mm, Höhe 100 cm.).

Nach Durchlauf von 500 ml des Carbonatpuffers wurde die Säure mit 1000 ml einer 2,5 %igen Ammoniaklösung in Wasser gewaschen.

Der erste Peak bei 200 ml Elutionsvolumen enthält PDE-Aktivitat*und Fluoreszenz.

Nicht umgesetzter Farbstoff und nicht umgesetztes PDE eluieren später.

**Patentansprüche**

**1.** Wasserlösliche polymere Farbstoffe der Formel (I)

$$[A]_a\text{-}[B]_b\text{-}[C]_c\text{-}[D]_d \qquad (I),$$

worin

- A einen gegebenenfalls vorhandenen wasserlöslich machenden Baustein,
- B ein über eine Ester-, Säureamid-, Urethan-, Harnstoff- und/oder Thioharnstoff-Gruppierung kovalent gebundenes Fluoreszenzfarbstoffmolekül,
- C einen zweiten Fluoreszenzfarbstoff, der über eine Ester-, Säureamid-, Urethan-, Harnstoff- und/oder Thioharnstoff-Gruppierung kovalent gebunden und nach Anregung ein Licht in einem Wellenlängenbereich emitiert, das um ± 30 nm von der Wellenlänge abweicht, bei der der jeweilige Fluoreszenzfarbstoff B seine maximale Absorption besitzt und
- D einen Monomerbaustein, der eine kovalente Verknüpfung zu einem Antikörper, Antigen, Hapten, einer Nucleinsäure oder einem geeignet funktionaliserten Oligonucleotid und gegebenenfalls zu den Komponenten B und/oder C ermöglicht,

bedeutet und

a,b,c und d die gew.-%igen Anteile der Komponenten A, B, C und D darstellen, die zusammen 100 Gew.-% ergeben.

* Prüfung auf PDE-Aktivität:
400 $\mu$l Eluat
600 $\mu$l Trispuffer pH 8,8/0,1 m
200 $\mu$l Mg-acetat 0,3 m
und 1000 $\mu$l Bis-p-nitrophenylphosphat/mmolar werden 105 Minuten bei 37°C reagiert, wobei Gelbfärbung sichtbar wird.

**2.** Verwendung von polymeren Farbstoffen gemäß Anspruch 1 zum markieren von biologischen Substanzen.

## Claims

**1.** Water-soluble polymeric dyestuffs of the formula (I)

$$[A]_a\text{-}[B]_b\text{-}[C]_c\text{-}[D]_d \qquad (I)$$

in which

A denotes an optionally present water-solubilising building block,

B denotes a fluorescent dyestuff molecule covalently bound via an ester, acid amide, urethane, urea and/or thiourea grouping,

C denotes a second fluorescent dyestuff which is covalently bound via an ester, acid-amide, urethane, urea and/or thiourea grouping and on excitation, emits light in a wavelength range which differs by ± 30 nm from the wavelength at which the particular fluorescent dyestuff B has its maximum absorption and

D denotes a monomeric building block which makes a covalent linkage to an antibody, antigene, haptene, a nucleic acid or a suitably funtionalised oligonucleotide and, if desired, to components B and/or C possible,

and

a, b, c and d represent the percentages by weight of components A, B, C and D, which together add up to 100 % by weight.

**2.** Use of polymer dyestuffs according to Claim 1 for labelling biological substances.

## Revendications

**1.** Colorants polymères solubles dans l'eau de formule I

$$[A]_a\text{-}[B]_b\text{-}[C]_c\text{-}[D]_d \qquad (I),$$

dans laquelle

A représente un constituant hydrosolubilisant éventuel,

B représente une molécule de colorant fluorescent liée par des liaisons covalentes par l'intermédiaire d'un groupement ester, amide, uréthane, urée et/ou thiourée,

C représente un deuxième colorant fluorescent relié par des liaisons covalentes par l'intermédiaire d'un groupement ester, amide, uréthane, urée et/ou thiourée et qui, après excitation, émet une lumière dans un intervalle de longueur d'ondes qui s'écarte de ± 30 nm de la longueur d'ondes à laquelle le colorant fluorescent particulier B a son maximum d'absorption et

D représente un constituant monomère qui permet une liaison par liaisons covalentes à un anticorps, un antigène, un haptène, un acide nucléique ou un oligonucléotide portant les fonctions appropriées et le cas échéant aux composants B et/ou C, et

a, b, c et d sont les proportions en % en poids des composants A, B, C et D, dont le total est de 100 % en poids.

**2.** Utilisation des colorants polymères selon la revendication 1 pour le marquage de substances biologiques.